# EUROPEAN PATENT SPECIFICATION

(11) **EP 1 187 375 B1**
(45) Date of publication and mention of the grant of the patent: **17.03.2004**
(21) Application number: 01121387.3
(22) Date of filing: 06.09.2001
(51) Int. Cl.: H04B 13/00, G07C 9/00

(54) **Data transmission system using a human body as a signal transmission path**
Datenübertragungssystem unter Verwendung eines menschlichen Körpers als Signalübertragungsweg
Système de transmission de données utilisant un corps humain comme voie de transmission de signaux

(30) Priority: 08.09.2000 JP 2000272984
(43) Date of publication of application: 13.03.2002
(73) Proprietor: Matsushita Electric Works, Ltd., Kadoma-shi, Osaka-fu 571-8686 (JP)
(72) Inventor: Doi, Kenji, c/o Matsushita Electric Works, Ltd., Kadoma-shi, Osaka 571-8686 (JP); Hashimoto, Masaru, c/o Matsushita Elec. Works, Ltd, Kadoma-shi, Osaka 571-8686 (JP); Koyama, Masaki, c/o Matsushita Electr. Works, Ltd., Kadoma-shi, Osaka 571-8686 (JP); Suzuki, Yoshiko, c/o Matsushita Electr. Works, Ltd, Kadoma-shi, Osaka 571-8686 (JP); Nishimura, Tokuhisa, c/o Matsushita El. Works, Ltd, Kadoma-shi, Osaka 571-8686 (JP)
(74) Representative: Appelt, Christian W.

(56) References cited:
- EP-A- 0 843 425
- WO-A-87/03119
- US-A- 4 591 854
- US-A- 5 811 897

## Description

### BACKGROUND OF THE INVENTION

The present invention relates to a data transmission system using a human body as a signal path, and more particularly to a system composed of a wearable transmitter, a receiver adapted to be connected to an associated equipment which utilize data transmitted from the transmitter, and a garment integrally holding two electrodes for passing the data through the human body.

EP application No. 00113564.9 discloses a data transmission system using the human body as a signal path. The system includes a portable transmitter in the form of a wrist watch to be worn on a user, and a signal receiver. The transmitter has a pair of electrodes on the back of the wrist watch for direct contact with the skin of the user. One electrode acts as a signal electrode which is connected through a portion of the user's body to a touch electrode of the signal receiver, while the other electrode acts as a ground electrode which is coupled through the other portion of the user's body to a circuit ground of the signal receiver to complete a signal path through the user's body for data transmission from the wrist watch to the signal receiver. When using this system used for a verified access to a place or database, however, the user is always required to keep in mind to carry the dedicated wrist watch having the electrodes. This may be sometimes inconvenient and even troublesome for the user who has his own wrist watch.

The European Patent Application EP 0 843 425 A2 further discloses also an electronic communication apparatus using the human body as a transmission medium, whereas the electronic communication apparatus comprises a portable transmitter to be carried or worn on a user's person, whereas the transmitter, being preferably in the form of a cart to be carried by the user, encloses both a top electrode and a bottom electrode as well as electronics and a micro processor.

### DISCLOSURE OF THE INVENTION

Based on the state of art, it is an object of the present invention to provide a data transmission system which is capable of assuring successful data transmission without requiring special attention to the user, especially assuring sufficient and reliable data transmission.

This object is solved by a data transmission system according to claim 1 and a transmitter according to claim 21, claims 2 to 20 refer to preferred embodiments of the data transmission system according to claim 1.

The system in accordance with the present invention comprises a transmitter adapted to be carried by the user and a receiver adapted to be connected to an associated equipment which utilizes data transmitted from the transmitter. The transmitter has a ground electrode to be placed in close proximity to the human body, a signal electrode to be placed also in close proximity to the human body in a spatially spaced relation from the ground electrode, a data memory storing first data, a first modulator for converting the first data into a first modulated voltage signal, and a first signal transmitter which applies the first modulated voltage signal across the signal electrode and the ground electrode. The receiver includes a circuit ground adapted to be connected to the ground, a touch electrode adapted for direct contact with a portion of the human body carrying the transmitter, a signal detector connected across the signal electrode and the circuit ground to detect the first modulated voltage signal, and a demodulator which converts the first modulated voltage signal back into the first data. The characterizing feature of the present invention resides in that the system includes a garment which is adapted to be worn by a user and integrates the ground and signal electrodes in such a manner that at least one of the electrodes is kept in a closely facing relation to the skin of the user and in a spaced relation with each other along a surface of said garment, thereby establishing an electrical path extending through a portion of the human body for signal transmission from the transmitter to the receiver. With the integration of the two electrodes into the garment, the user wearing the garment as an everyday clothes or uniform such as a white gown can be easy and convenient to carry the transmitter for successful transmission of the data to the receiver.

Preferably, each of the ground and signal electrodes is formed by a plurality of electrically conductive threads and is sewed to be integrated into the garment. Thus, the electrodes can be easily integrated into the garment and cannot sacrifice comfortableness of the garment. Each electrode made of the electrically conductive threads can be woven into a fabric so as to be lined on the garment. Alternatively, the electrode of the conductive threads can be woven into an indispensable part of the garment. With the use of the electrical conductive threads, the garment provided with the resulting electrodes can be washed like ordinary clothes, which enhances availability of the system.

The ground electrode is preferred to be located on the garment closer to the foot of the user than the signal electrode for establishing a consistent electrical path through the human body. That is, the electrical path is composed of a first fraction path extending from the ground electrode down to the foot of the user and through the ground to the circuit ground of the receiver, and a second fraction path extending from the signal electrode towards and through a finger of the user to the touch electrode of the receiver without interfering the first fraction path, thereby assuring efficient and reliable data transmission.

In a preferred embodiment where both of the ground and signal electrodes are held on the garment so as to come into a closely facing relation with the skin of the user, the transmitter has a case which accommodates an electrical circuitry realizing the first modulator and the first signal transmitter, and which is formed as a separate article from the electrodes. The case is provided with terminals for electrically connecting the circuitry with the ground and signal electrodes. In this connection, the garment is additionally provided with a ground lead and a signal lead which extend respectively from the ground and signal electrodes for connection with the terminals of the case. Both of the ground and signal leads are formed by a strand of the electrically conductive threads and are sewed on the garment. Thus, the leads can be also easily and consistently integrated into the garment to retain comfortableness of the garment.

A coupling member is included in the system to make the case detachable from the garment and at the same time make the electrical circuitry detachable from the electrodes, i.e., the corresponding leads. The coupling member may be realized by a spring-loaded clip which is pivotally supported to the case so as to be movable between a pinching position and a release position. The clip is formed with the terminals which are electrically isolated from each other for connection respectively with the ground and signal leads at the pinching position.

Instead of the clip, the coupling member may comprise a pair of first fasteners each composed of one of a socket and a ball forming a snap button for mounting the case to the garment, and a pair of second fasteners each composed of the other of the socket and the ball. The first fasteners are fixed on the case and connected across the first signal transmitter of the circuitry, while the second fasteners are fixed on the garment and are permanently connected to respectively to the ground and signal electrodes. When using the snap button to make the case detachable from the garment, the second fasteners are preferably held in direct contact with the ground and signal electrodes, respectively formed of the electrically conductive threads, thereby substantially eliminating the leads from the garment. In this connection, the ground and signal electrodes may be in the form of annular bands provided inside of a sleeve of the garment in a spaced relation from each other along the length of the sleeve.

Preferably, the case is made water-tight for sealing the electric circuitry so that the garment can be washed like ordinal clothes even with the case. Further, the case may be in the form of a plate which encapsulate the circuitry and a battery energizing the circuitry. Thus, the plate can be utilized also as a nameplate as is usual with the white gown worn by a physician, nurse, and a laboratory worker.

These and still other objects and advantageous features will become more apparent from the following description of the preferred embodiments when taken in conjunction with the attached drawings.

### BRIEF DESCRIPTION OF THE DRAWINGS

FIG. 1 is a schematic view illustrating a basic concept of a data transmission system in accordance with the present invention;
FIG. 2 is a block diagram of a wearable transmitter utilized in the above system;
FIG 3 is a block diagram of an associated receiver utilized in the above system;
FIGS. 4 and 5 are front and rear views of a garment utilized in the above system to integrate a ground electrode and a signal electrode;
FIG. 6 is a perspective view showing one typical application of the above system;
FIG. 7 is a rear perspective view of a case in the form of a nameplate accommodating an electric circuitry of the transmitter and detachable to the garment;
FIG. 8 is a perspective view of the case and a portion of the garment to which the case is attached;
FIG. 9 is a view showing the nameplate as attached to the garment;
FIG 10 is schematic view illustrating another embodiment of the system;
FIG. 11 is an exploded perspective view showing ground and signal electrodes integrated into a sleeve of the garment and a transmitter case detachable thereto; and
FIG. 12 is a side view of the transmitter case.

### DETAILED DESCRIPTION OF THE EMBODIMENTS

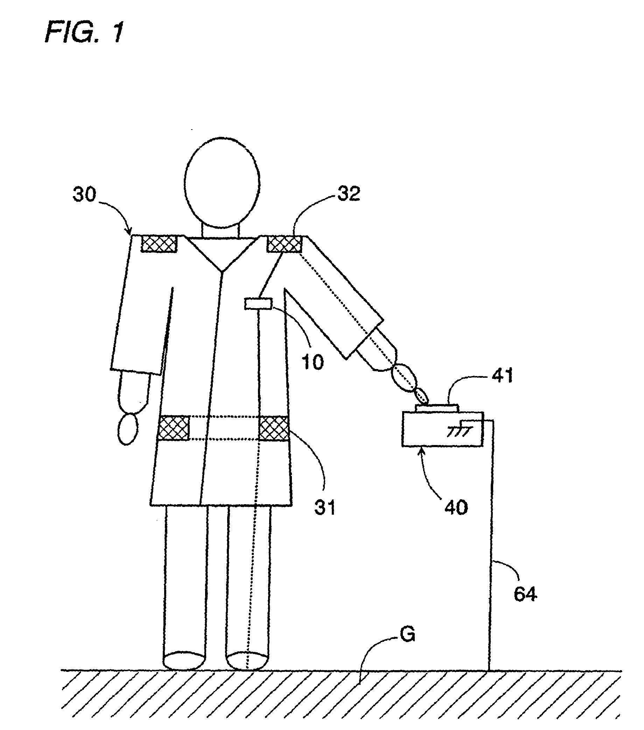

Referring first to FIG 1, there is shown a principle of a data transmission system using a human body as a signal transmission path. The system includes a wearable transmitter **10** adapted to be worn on the human body, and a receiver **40** adapted to be connected to an equipment such as a personal computer **60** which utilizes data transmitted from the transmitter for controlled operation of the computer **60**, for example, a verified log-in of the user. The transmitter **10** is connected to a ground electrode **31** and a signal electrode **32** which are integrated into a garment **30** worn by a user in close proximity to the skin of the user. When the user carrying the transmitter **10** touches a touch electrode **41** of the receiver **40,** a signal path is established which extends from the signal electrode 32 through a portion of the user's body, the touch electrode **41**, an internal circuit of the receiver **40**, a circuit ground **49** of the receiver **40**, the ground G, the other portion of the user's body, the ground electrode **31** and an internal circuit of the transmitter **10**. The signal path extending through the human body is indicated by dotted lines. Thus, a voltage signal applied across the electrodes **31** and **32** is transmitted to the receiver **40** when the user touches the touch electrode **41.** In FIG. 1, the circuit ground of the receiver **40** is connected to the ground **G** through a ground line **64** common to the computer **60** for the sake of simplicity. However, the circuit ground may be capacitively connected to the ground G or even capacitively connected directly to the major portion of the user's body for establishing the signal path.

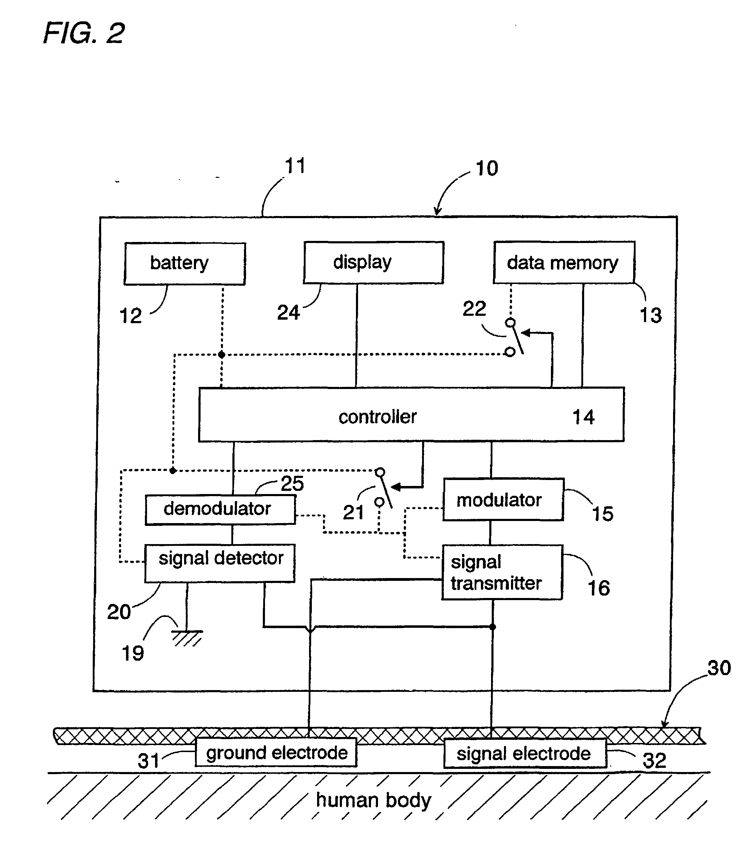

As shown in FIG. 2, the transmitter **10** includes an electric circuitry and a battery 12 which are accommodated within a case **11.** The circuitry includes a data memory **13** storing data to be transmitted, a controller **14**, a modulator **15** modulating the data into a modulated voltage signal, a signal transmitter **16** applying the modulated voltage signal across the signal electrode **32** and the ground electrode **31** on the garment **30**. Also included in the circuitry is a signal detector **20** which is connected to detect a start signal transmitted from the receiver **40** through the signal electrode **32**. The start signal is received across the signal electrode **32** and a circuit ground **19.** The circuit ground **19** may be connected to the ground electrode **31.** Only the controller **14** and the signal detector **20** are constantly energized by the battery **12** to be ready for detecting the start signal from the receiver **40**. In the non-operative condition where the transmitter **10** is not transmitting the data, the controller **14** is kept in a sleep mode of consuming less electric current from the battery **12**. When the start signal is received as a consequence of the user touching the touch electrode **41,** the signal detector **20** wakes up the controller **14** which in turn energizes the data memory **13,** the modulator **15,** and the signal transmitter **16** to apply the modulated voltage signal across the signal electrode **32** and the ground electrode **31** for initiating the data transmission. The controller **14** incorporates a timer which starts upon detection of the start signal to provide a predetermined time during which the data is transmitted. After the elapse of the predetermined time, the controller **14** responds to deenergize the modulator **15,** the signal transmitter **16** and the data memory **13**. For this purpose, the controller **14** includes power switches **21** and **22** which are actuated by the signal detector **20** and the timer to selectively energize and deenergize the modulator **15**, the signal transmitter 16 and the data memory **13**. Dotted lines in FIG 2 show power supply lines from the battery **12**. Thus, after transmitting the data, the controller **14** goes back into the sleep mode of consuming less current or energy but being kept ready to detect of the start signal for another data transmission. The transmitter **10** optionally includes a display **24** for visual indication of the data stored in the data memory **15.**

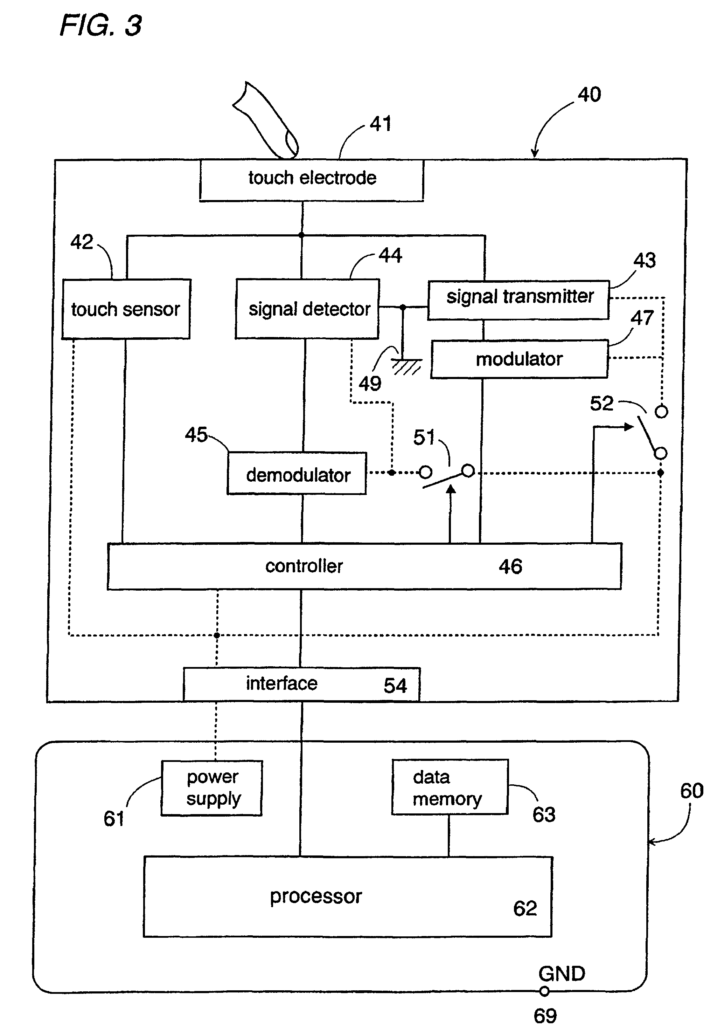

As shown in FIG. 3, the receiver **40** includes various circuits connected to the touch electrode **41** on the exterior of a housing of the receiver. The circuits are energized by a power source **61** provided in the computer **60** to which the receiver **40** is attached. The circuits are commonly connected to a circuit ground **49** which is in turn connected to a ground terminal **69** of the computer for connection with the ground. The circuits include a touch sensor **42** which is connected to the touch electrode **41** to give a touch signal when the touch electrode **41** is touched by the user's body. Also included in the circuits are a start signal generator **43,** a signal detector **44,** a demodulator **45,** and a controller **46** which controls the operations of the circuits. The signal transmitter **43** applies the start signal to the touch electrode **41** in response to the touch signal. The signal detector **44** detects the modulated voltage signal which is transmitted from the transmitter **10** and received across the touch electrode **41** and the circuit ground **49**. The modulated voltage signal thus detected is demodulated at the demodulator **45** to derive the first data which is then fed to the computer **60** to be processed thereat. For example, the first data includes a user's identification code which is verified at a processor **62** of the computer with reference to various codes assigned to different users and stored in a data memory **63**. When the user's ID is verified as a correct one, the computer completes the log-in sequence to permit the access by the user.

Under the non-operating condition where the touch electrode **41** is not touched by the human body, only the controller **46** and the touch sensor **42** are energized to be ready for detection of the touching. Upon the touch electrode **41** being touched, the touch sensor **42** gives the touch signal to the controller **46** which responds to close switches **51** and **52** to energize the signal transmitter **43,** the signal detector **44,** and the demodulator **45,** thereby generating the start signal and making the circuits ready for receiving the data from the transmitter **10**. The controller **46** also includes a timer which starts, upon receiving the touch signal, to provide a predetermined time interval during which the data transmission from the first transceiver **10** is expected to complete. After the elapse of the predetermined time interval, the controller **46** responds to open the switches **51** and **52**, deenergizing the signal transmitter **43**, the signal detector **44**, and the demodulator **45**. Thus, the receiver **40** is kept in a sleep mode of consuming less electricity until the touch electrode **41** is touched. Dotted lines in FIG. 3 show power supply lines. The receiver **40** further includes an interface **54** in the form of the USB for transferring the data to the computer **60** as well as for receiving the power from a power supply **61**.

Further, the transmitter **10** and the receiver **40** are designed to effect a bilateral data transmission therebetween. For this purpose, the transmitter **10** additionally includes a demodulator **25** for demodulating data transmitted from the receiver **40** and that the receiver **40** additionally includes a modulator **47** for modulating the data to be transmitted from the receiver **40**. The modulator **47** of gives a modulated voltage signal indicative of the data to be transmitted to the transmitter **10**. The signal transmitter **43** of the receiver **40** is responsible for applying the modulated voltage signal to the touch electrode **41** for data transmission back to the transmitter **10**.

In operation, when the user touches the touch electrode **41** of the receiver **40**, the touch sensor **42** provides a touch signal in response to which the controller **46** energizes the modulator **47**, the signal transmitter 43, the demodulator **45,** and the signal detector **44.** At first, the controller **46** retrieves the data from the data memory **63** of the computer **60** and instructs to give and apply the modulated voltage signal indicative of the data. In response to the voltage signal from the receiver **40**, the controller **14** of the transmitter **10** activates the data memory **13** and performs a suitable processing of the data from the data memory **13** in consideration of the data received from the receiver **40**. The controller **14** updates the data of the data memory **13** depending upon the result of the processing. Thereafter, the controller **14** activates the modulator **15** and the signal transmitter **16** so as to transmit the modulated voltage signal indicative of the updated data to the receiver **40** through the electrodes **31** and **32**. The modulated voltage signal received at the receiver **40** is converted into the data which is utilized by the controller **46** for a controlled operation of the computer or passed to another equipment to be processed thereat for a specific operation of the equipment. In this manner, the two-way data transmission is made between the transmitter and the receiver in a half-duplex manner. Depending upon a specific application to which the system is applied, the system may be designed to have more than one data transmission cycles in which the one-way data transmission from either of the transmitter and the receiver repeats twice or more. In such case, the data in the data memory **13** of the transmitter **10** is modified or updated by the data transmitted from the receiver **40**.

Also, for minimizing energy consumption, the transmitter **10** is kept in the sleep mode until the modulated voltage signal is received from the receiver **40**, and comes back again in the sleep mode after the data transmission between the transmitter and the receiver is completed. In other words, the data memory **13**, the modulator **15**, the signal transmitter **16**, and the demodulator **21** are energized by closure of the switches **21** and **22** only for a predetermined time period starting from receiving the modulated voltage signal from the receiver. It is within the predetermined time period that the data transmission between the transmitter and the receiver is completed. Likewise, the receiver is kept in the sleep mode until the touch electrode **41** is touched by the human body, and come back to the sleep mode after the data transmission between the first and second transceivers are completed. Thus, the signal transmitter **43**, the modulator **47**, the signal detector **44**, and the demodulator **45** are energized by closure of switches **51** and **52** only for a predetermined time period starting from the touch electrode being touched.

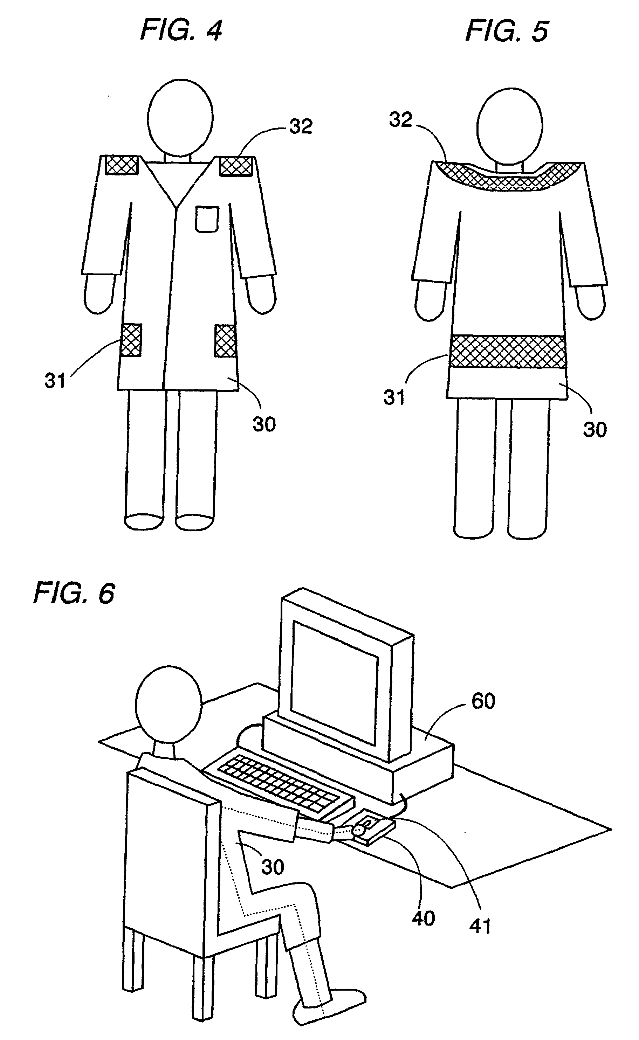

As shown in FIGS. 1, 4, and 5, the garment **30** to which the electrodes **31** and **32** are attached is selected, for example, as a white gown that is always worn by a particular user like a physician, nurse, and laboratory worker while engaging a job requiring a verification of the user. As a matter of course, the garment **30** is not limited to the white gown and may take various types of the clothes such as a uniform for an office, factory, school, and the like organization or group. Each of the ground electrode **31** and the signal electrode **32** is in the form of a fabric made by electrically conductive threads and is sewed on the inner surface of the garment **30** with the signal electrode **32** disposed at the shoulders of the garment **30** and with the ground electrode 31 disposed around the lower part of the garment corresponding to a hip and buttocks of the user, as shown in FIGS. 4 and 5. Instead of being lined on the garment, the electrodes may be woven into the garment as indispensable parts thereof. The above selected location of the electrodes **31** and **32** is particularly effective when the user access the computer **60** while sitting on a chair as shown in FIG. 6. In this condition, the ground electrode **31** receives a counter force from the seat of the chair to be pressed against the buttocks of the user, while the signal electrode **32** is pressed against the shoulders of the user with the help of weight of the garment for reliable electrical connection of the electrodes to the human body. It is noted in this connection that the ground electrode **31** is located closer to the foot of the user than the signal electrode **32** along the signal path extending through the human body so that the path extending from the signal electrode **32** toward the finger of the user can be substantially free from, i.e., cannot be substantially interfered with the path extending from the ground electrode **31** to the foot of the user for reliable signal transmission between the transmitter **10** and the receiver **40**.

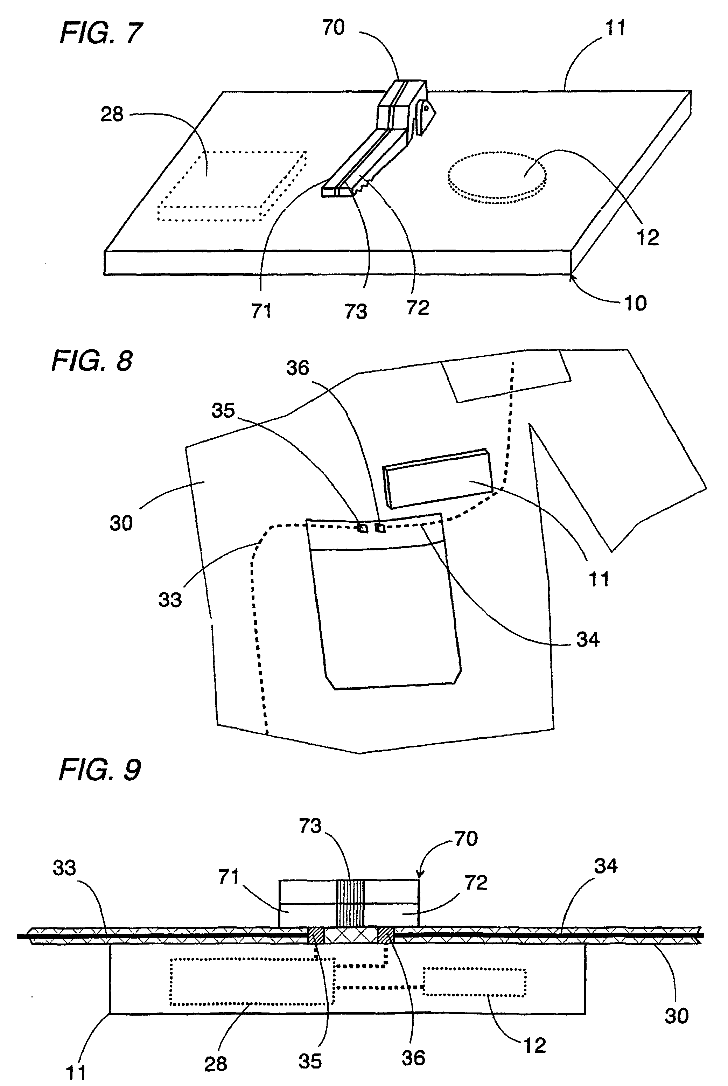

As shown in FIG. 7, the case **11** of the transmitter **10** is formed into a nameplate which is made water-tight and accommodates therein the electric circuitry **28** forming the various functional circuits and elements as shown in FIG 2, and the battery **12** energizing the circuits. The case **11** is provided with a spring-loaded clip **70** so as to be detachable to the garment, for example, at a breast pocket. The clip **70** is pivotally supported at its one end to the case so as to be movable between a pinching position and a release position. The clip **70** includes a pair of conductive terminals **71** and **72** which are connected to the electric circuitry, i.e., across the signal transmitter **16** and which are isolated by a dielectric strip **73**. As shown in FIGS. 8 and 9, when the case **11** is attached to the garment, i.e., the breast pocket, the terminals **71** and **72** come into engagement respectively with pads **35** and **36** provided at one ends of respective leads **33** and 34 extending from the individual electrodes **31** and **32**. Thus, the electric circuitry of the transmitter is connected to electrodes. In this connection, the leads **33** and **34** are also made of electrically conductive threads, more particularly, strands of the conductive threads sewed on or into the garment **30.**

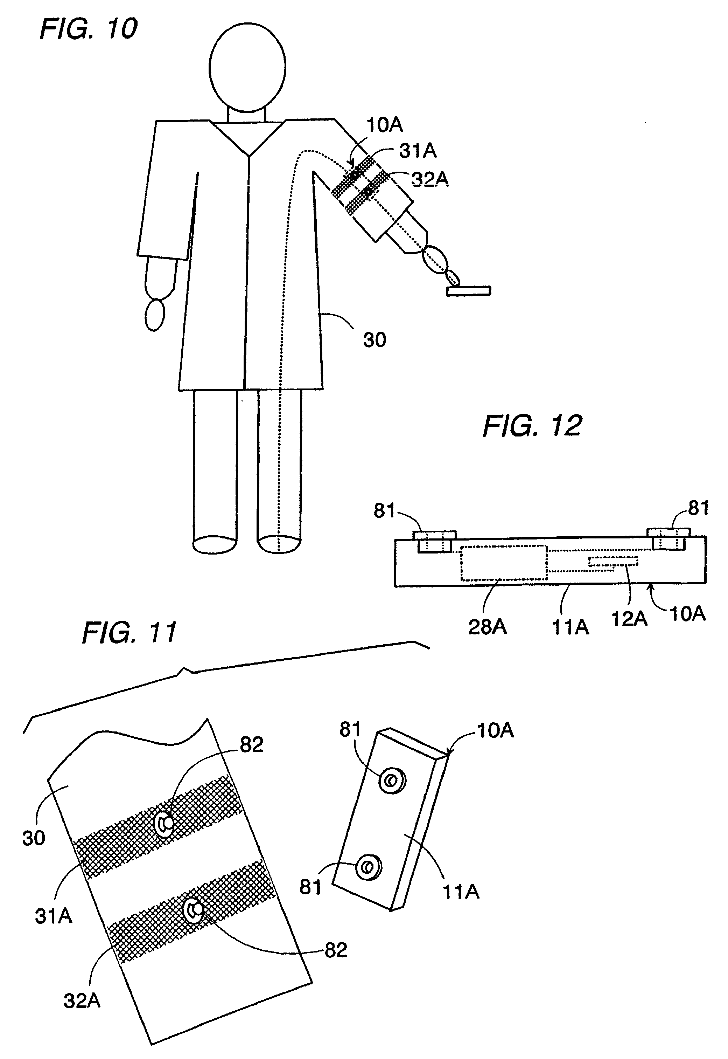

FIG. 10 to 12 show another preferred embodiment of the present invention in which a case **11A** of the transmitter **10A** is detachable to a sleeve of the garment **30** by means of snap buttons which are normally utilized in association with clothing. That is, the snap button is made of conductive material and composed of a socket **81** and a ball **82**. In this connection, a ground electrode **31** A and a signal electrode **32A** are provided at the sleeve of the garment **30** for direct coupling with the electric circuitry of the transmitter **10A.** Other structures are identical to the above embodiment and therefore no duplicated explanation is made herein. The case **11A** is in the form of a water-tight thin plate accommodating the electric circuitry **28A** of the transmitter **10A** and the battery **12A**. The case **11A** is provided with a pair of the sockets **81** which are internally connected to the electrical circuitry of the transmitter **10A**, while the electrodes **31 A** and **32A** are provided respectively with the balls **82**. As in the previous embodiment, each electrode is made of conductive threads woven and sewed on or into the sleeve to form an annular band surrounding the sleeve in close proximity to the skin of the user wearing the garment for establishing a reliable electrical connection to the human body. In this embodiment, the balls **82** are sewed directly on the electrodes by use of the conductive threads or press-fitted into the electrode, thereby eliminating the leads extending otherwise by a certain distance along the garment from the electrodes. Alternatively, the balls may be provided on the case, or a mixed pair of the ball and socket is provide on the case.

In the illustrated embodiments, the ground and signal electrodes **31** and **32** are explained to be formed by the electrically conductive threads, however, the each electrode may be formed as a metal plating deposited on the surface of the garment or deposited on a fabric which is sewed on the garment. Also, it is noted that the garment into which the electrodes are integrated is not limited to the garment like the white gown and may be any other kinds of the clothing that is constantly worn by the user who is in access to the verified system. Therefore, the clothing may include an armband and wristband integrating the electrodes to which the case of the transmitter can be made electrically and physically detachable by use of the above described snap buttons.

Further, the illustrated embodiments show only one application where both of the electrodes are kept in close facing relation with the skin of the user so that both of the electrodes are in direct electrical connection to the user's body, however, it is equally possible that one of the electrodes is in direct facing relation, i.e., electrical connection to the user's body, while the other of the electrodes is arranged to face away from the user's skin for capacitive connection to the receiver through the air.

Still further, although the illustrated embodiment is arranged to verify the data, i.e., the user's ID at the computer **60**, the receiver **40** may be arranged to equip the processor and the data memory so as to have a function of verifying the data from the transmitter, and providing a verified output to an associated device for permitting the access or a required control of the device, for example, permitting an entry of the user into a restricted area.

The features disclosed in the foregoing description, in the claims and/or in the accompanying drawings may, both separately and in any combination thereof, be material for realising the invention in diverse forms thereof.

### LIST OF REFERENCE NUMERALS

- 10: transmitter
- 11: case
- 12: battery
- 13: data memory
- 14: controller
- 15: modulator
- 16: signal transmitter
- 19: circuit ground

- 20: signal detector
- 21: switch
- 22: switch
- 24: display
- 25: demodulator
- 28: electric circuitry

- 30: garment
- 31: ground electrode
- 32: signal electrode
- 33: lead
- 34: lead
- 35: pad
- 36: pad

- 40: receiver
- 41: touch electrode
- 42: touch sensor
- 43: signal transmitter
- 44: signal detector
- 45: demodulator
- 46: controller
- 47: modulator

- 51: switch
- 52: switch
- 54: interface

- 60: computer
- 61: power supply
- 62: processor
- 63: data memory
- 64: ground line
- 69: ground terminal

- 70: clip
- 71: terminal
- 72: terminal
- 73: dielectric strip

- 81: socket
- 82: ball

## Claims

1. A data transmission system using a human body as a signal transmission path, said system comprising a transmitter (10) adapted to be worn on a human body, and a receiver (40) adapted to be connected to an associated device which utilizes data transmitted from the transmitter, said transmitter (10) comprising:
- a ground electrode (31;31A) and a signal electrode (32;32A) which are placed in close proximity to the human body; and
- a data memory (13)for storing first data to be transmitted;
- a first modulator (15) for converting said first data into a first modulated voltage signal; and
- a first signal transmitter (16) which applies the first modulated voltage signal across said signal electrode and said ground electrode;
said receiver (40) comprising:
- a circuit ground (49) adapted to be connected to the ground ;
- a touch electrode (41) adapted for direct contact with a portion of the human body carrying said transmitter;
- a signal detector (44) connected across said touch electrode and said circuit ground to detect said first modulated voltage signal; and
- a demodulator (45) for converting said first modulated voltage signal back into said first data;
**characterized in that**
said system including a garment (30) which is adapted to be worn by a user and integrates said ground and signal electrodes (31, 32; 31 A, 32A) to establish an electrical path extending through a portion of the human body for signal transmission from said transmitter to said receiver, wherein both said ground electrode (31; 31A) and said signal electrode (32; 32A) are disposed in a closely facing relation to the skin of the user and in a spaced relation with each other along a surface of said garment (30).

2. The data transmission system as set forth in claim 1, wherein
both of said ground and signal electrodes (31, 32; 31 A, 32A) are carried on the garment so as to come into a closely facing relation with the skin of the user,

3. The data transmission system as set forth in claim 1, wherein
each of said ground and signal electrodes (31, 32; 31A, 32A) is formed by a plurality of electrically conductive threads and is sewed to be integrated into the garment.

4. The data transmission as set forth in claim 3, wherein
each of said ground and signal electrodes (31, 32; 31 A, 32A) is provided in the form of a fabric made of the electrically conductive threads and is lined on said garment.

5. The data transmission system as set forth in claim 3,
wherein each of said ground and signal electrodes (31, 32; 31A, 32A) is merged into said garment to constitute an indispensable part of the garment.

6. The data transmission system as set forth in claim 1,
wherein each of said ground and signal electrodes (31, 32; 31 A, 32A) is formed by a metal plating deposited on the surface of the garment.

7. The data transmission system as set forth in claim 1,
wherein each of said ground and signal electrodes is (31, 32; 31 A, 32A) formed by a metal plating deposited on a fabric which is sewed on the garment.

8. The data transmission system as set forth in claim 2, wherein
said ground electrode (31; 31 A) is located on said garment closer to the foot of the user than said signal electrode (32; 32A) along said electrical path extending through the portion of the human body.

9. The data transmission system as set forth in claim 8, wherein
said signal electrode (32) is located on a shoulder of the garment.

10. The data transmission system as set forth in claim 3, wherein
both of said ground and signal electrodes (31, 32; 31A, 32A) are carried on the garment so as to come into a closely facing relation with the skin of the user,
said transmitter including a case (11) which accommodates an electrical circuitry (28) realizing said first modulator (15) and said first signal transmitter (16) and which is formed separately from said ground and signal electrodes, said case being provided with terminals (71, 72) for electrically connecting said electric circuitry with said ground and signal electrodes,
said garment carrying a ground lead (33) and a signal lead (34) which extend respectively from said ground and signal electrodes for connection with said terminals of said case, each said ground and signal leads being formed by a strand of electrically conductive threads and sewed on said garment.

11. The data transmission system as set forth in claim 10, wherein
said system includes a coupling means (70) by which said case (11) is detachably connected to said garment and at the same time said electrically circuitry is detachably connected to said ground and signal electrodes (31, 32),
said coupling means comprising a spring-loaded clip (70) which is pivotally supported to said case to be movable between a pinching position and a release position, said clip being provided with said terminals (71, 72) which are electrically isolated from each other for connection respectively with said ground and signal leads (33, 34) at said pinching position.

12. The data transmission system as set forth in claim 2, wherein
said transmitter (10) including a case (10; 11A) which accommodates an electric circuitry (28) realizing said first modulator (15) and said first signal transmitter (16) and which is formed separately from said ground and signal electrodes, said case being provided with terminals (71, 72; 81) for electrical connecting said electric circuitry respectively with said ground and signal electrodes,
said system including a coupling means (70; 81, 82) by which said case is detachably connected to said garment and at the same time said electrical circuitry is connected to said ground and signal electrodes.

13. The data transmission system as set forth in claim 12, wherein
said coupling means comprises a pair of first fasteners each composed of one of a socket (81) and a ball (82) forming a snap button for mounting said case (11A) to the garment (30), a pair of second fasteners each composed of the other of the socket and ball, said first fasteners (81) being fixed on said case and connected across said first signal transmitter, and said second fasteners (82) being fixed on said garment and permanently connected respectively to said ground and signal electrodes.

14. The data transmission system as set forth in claim 13, wherein
each of said ground and signal electrodes is formed by a plurality of electrically conductive threads sewed and integrated into the garment, and said second fasteners (82) are held in direct contact with said ground and signal electrodes (31 A, 32A), respectively.

15. The data transmission system as set forth in claim 14, wherein
said ground and signal electrodes (31 A, 32A) are respectively in the form of annular bands provided around a sleeve of the garment in a spaced relation from each other along the length of the sleeve.

16. The data transmission system as set forth in claim 12, wherein
said case (11; 11 A) is made water-tight for sealing said electrical circuitry.

17. The data transmission system as set forth in claim 1,
wherein said case (11; 11A) is provided in the form of a plate which encapsulates an electrical circuitry (28) realizing said first modulator (15) and said first signal transmitter (16), and also a battery (12) energizing the circuitry.

18. The data transmission system as set forth in claim 1, wherein
said receiver (40) additionally includes a second modulator (47) for converting second data into a second modulated voltage signal which is applied between said touch electrode (41) and said circuit ground (49);
said transmitter (10) further including a first demodulator (25) for converting said second modulated voltage signal, which is detected through said signal electrode (32; 32A), into said second data.

19. The data transmission system as set forth in claim 18, wherein
said transmitter (10) includes a controller (14) which has a function of modifying said first data in accordance with said second data.

20. The data transmission system as set forth in claim 1, wherein
said first data includes a single user's identification code (ID) to be transmitted to said receiver, and said receiver includes an ID memory storing a plurality of assigned user's identification codes respectively given to a plurality of users, and a verifier which verifies the user's ID in comparison with the plurality of the assigned user's identification codes so as to permit an intended action requested by the user wearing said garment.

21. A transmitter (10) for a data transmission system using a human body as a signal transmission path, said transmitter (10) being adapted to be worn on a human body and comprising:
- a ground electrode (31; 31A)and a signal electrode (32; 32A) which are placed in close proximity to the human body;
- a data memory (13) for storing first data to be transmitted;
- a first modulator (15) for converting said first data into a first modulated voltage signal; and
- a first signal transmitter (16) which applies the first modulated voltage signal across said signal electrode and said ground electrode;
**characterized in that** said ground and signal electrodes (31, 32; 31A, 32A) are integrated in a garment (30) which is adapted to be worn by a user to establish an electric path extending through a portion of the human body for signal transmission from said transmitter (10) to a receiver of said data transmission system, wherein both said ground electrode (31; 31A) and said signal electrode (32, 32A) are disposed in a closely facing relation to the skin of the user and in a spaced relation with each other along a surface of said garment (30).

## Patentansprüche

1. Datenübertragungssystem, das einen menschlichen Körper als Signalübertragungspfad nutzt, wobei dieses System eine Sendevorrichtung (10), die so ausgelegt ist, daß sie an einen menschlichen Körper getragen werden kann, und eine Empfangsvorrichtung (40) umfaßt, die so ausgelegt ist, daß sie mit einer zugehörigen Vorrichtung verbunden werden kann, die Daten verwendet, die von der Sendevorrichtung übertragen werden, wobei die Sendevorrichtung (10) umfaßt:
- Eine Erdungselektrode (31; 31A) und eine Signalelektrode (32; 32A), die in unmittelbarer Nähe des menschlichen Körpers angeordnet werden;
- einen Datenspeicher (13) zum Speichern erster zu übertragender Daten;
- eine erste Modulationsvorrichtung (15) zum Umwandeln der ersten Daten in ein erstes moduliertes Spannungssignal; und
- eine erste Signalsendevorrichtung (16), die das erste modulierte Spannungssignal über die Signalelektrode und die Erdungselektrode anlegt;
wobei die Empfangsvorrichtung (40) umfaßt:
- Eine Schaltungserdung (49), die so ausgelegt ist, daß sie mit Masse verbunden werden kann;
- eine Berührungselektrode (41), die für einen direkten Kontakt mit einem Bereich des menschlichen Körpers, der die Sendevorrichtung trägt, ausgelegt ist;
- einen Signaldetektor (44), der über die Berührungselektrode und die Schaltungserdung verbunden ist, um das erste modulierte Spannungssignal zu detektieren;
- einen Demodulator (45) zum Umwandeln des ersten modulierten Spannungssignals zurück in die ersten Daten,
**dadurch gekennzeichnet, daß**
das System ein Kleidungsstück (30) umfaßt, das so ausgelegt ist, daß es von einem Benutzer getragen werden kann und die Erdungs- und die Signalelektrode (31, 32; 31A, 32A) aufnimmt, so daß ein elektrischer Pfad hergestellt wird, der sich durch einen Bereich des menschlichen Körpers für eine Signalübertragung von der Sendevorrichtung zu der Empfangsvorrichtung erstreckt, wobei sowohl die Erdungselektrode (31; 31A) als auch die Signalelektrode (32; 32A) der Haut des Benutzers direkt gegenüberliegend und längs einer Oberfläche des Kleidungsstücks (30) mit Abstand voneinander angeordnet sind.

2. Datenübertragungssystem nach Anspruch 1, wobei sowohl die Erdungs- als auch die Signalelektrode (31, 32; 31A, 32A) an dem Kleidungsstück gehalten sind, so daß sie der Haut des Benutzers direkt gegenüberliegend angeordnet sind.

3. Datenübertragsungssystem nach Anspruch 1, wobei sowohl die Grund- als auch die Signalelektrode (31, 32; 31A, 32A) durch eine Vielzahl von elektrisch leitenden Fäden gebildet sind und so vernäht sind, daß sie in dem Kleidungsstück integriert sind.

4. Datenübertragungssystem nach Anspruch 3, wobei sowohl die Erdungs- als auch die Signalelektrode (31, 32; 31A, 32A) in Form eines Gewebes zur Verfügung gestellt sind, das aus elektrisch leitenden Fäden hergestellt ist und auf dem Kleidungsstück aufgebracht ist.

5. Datenübertragungssystem nach Anspruch 3, wobei sowohl die Erdungs- als auch die Signalelektrode (31, 32; 31A, 32A) in das Kleidungsstück eingebracht ist, so daß es ein nicht lösbares Teil des Kleidungsstücks bildet.

6. Datenübertragungssytem nach Anspruch 1, wobei sowohl die Erdungs- als auch die Signalelektrode (31, 32; 31A, 32A) durch eine Metalloberfläche gebildet wird, die auf der Oberfläche des Kleidungsstücks aufgebracht ist.

7. Datenübertragunssytem nach Anspruch 1, wobei sowohl die Erdungs- als auch die Signalelektrode (31, 32; 31A, 32A) durch eine Metalloberfläche gebildet ist, die auf einem Gewebe aufgebracht ist, welches an dem Kleidungsstück angenäht ist.

8. Datenübertragunssystem nach Anspruch 2, wobei die Erdungselektrode (31; 31A) längs des elektrischen Pfades, der sich durch den Bereich des menschlichen Körpers erstreckt, näher an dem Fuß des Benutzers auf dem Kleidungsstück angebracht ist als die Signalelektrode (32; 32A).

9. Datenübertragunssystem nach Anspruch 8, wobei die Signalelektrode (32) auf einer Schulter des Kleidungsstückes angeordnet ist.

10. Datenübertragunsgerät nach Anspruch 3, wobei
- sowohl die Erdungs- als auch die Signalelektrode (31, 32; 31A, 32A) auf dem Kleidungsstück gehalten sind, so daß sie mit der Haut des Benutzers direkt gegenüberliegend angeordnet werden,
- wobei die Sendevorrichtung ein Gehäuse (11) umfaßt, das einen elektrischen Schaltkreis (28) aufnimmt, der den ersten Modulator (15) und die erste Signalübertragungsvorrichtung (16) realisiert, und das getrennt von der Erdungs- und der Signalelektrode ausgebildet ist, wobei das Gehäuse mit Anschlüssen (71, 72) für ein elektrisches Verbinden des elektrischen Schaltkreises mit der Erdungs- und der Signalelektrode versehen ist;
- wobei das Kleidungsstück einen Erdungsanschluß (33) und einen Signalanschluß (34) aufweist, die sich von der Grundelektrode einerseits und der Signalelektrode andererseits für einen Verbinden der Anschlüsse des Gehäuses erstrecken, wobei sowohl der Erdungs- als auch der Signalanschluß durch einen Strang elektrisch leitender Fäden gebildet und an dem Kleidungsstück angenäht sind.

11. Datenübertragungssystem nach Anspruch 10, wobei
- das System ein Kopplungsmittel (70) umfaßt, mit dem das Gehäuse (11) lösbar mit dem Kleidungsstück verbunden ist und gleichzeitig der elektrische Schaltkreis lösbar mit der Erdungs- und der Signalelektrode (31, 32) verbunden ist;
- das Kopplungsmittel einen federbelastete Klemme (70) umfaßt, die schwenkbar an dem Gehäuse gehalten wird, so daß sie zwischen einer Klemmposition und einer Löseposition bewegbar ist, wobei die Klemme mit den Anschlüssen (71, 72) versehen ist, die für die jeweilige Verbindung mit dem Erdungs- und dem Signalanschluß (33, 34) in der Klemmposition elektrisch voneinander isoliert sind.

12. Datenübertragungssystem nach Anspruch 2, wobei
- die Sendevorrichtung (10) ein Gehäuse (10; 11A) umfaßt, das einen elektrischen Schaltkreis (28) aufnimmt, der den ersten Modulator (15) und die erste Signalübertragungsvorrichtung (16) realisiert, und das getrennt von der Erdungs- und der Signalelektrode ausgebildet ist, wobei das Gehäuse mit Anschlüssen (71, 72; 81) zum elektrischen Verbinden des elektrischen Schaltkreises mit der Erdungs- und der Signalelektrode versehen ist;
- wobei das System ein Kopplungsmittel (70; 81, 82) umfaßt, mit dem das Gehäuse lösbar mit dem Kleidungsstück verbunden und zur gleichen Zeit der elektrische Schaltkreis mit der Erdungs- und der Signalelektrode verbunden ist.

13. Datenübertragungssystem nach Anspruch 12, wobei das Kopplungsmittel ein Paar erster Befestigungselemente, von denen jedes eine Aufnahme (81) oder ein Kugelelement (82) umfaßt, die einen Druckknopf zur Befestigung des Gehäuses (11A) an dem Kleidungsstück (30) bilden, und ein Paar zweiter Befestigungselemente umfaßt, die aus dem jeweils zugehörigen Element Aufnahme oder Kugelelement bestehen, wobei die ersten Befestigungselemente (81) an dem Gehäuse befestigt und über die erste Signalübertragungsvorrichtung verbunden sind und die zweiten Befestigungselemente an dem Kleidungsstück und permanent mit der Erdungs- bzw. der Signalelektrode verbunden sind.

14. Datenübertragungssystem nach Anspruch 13, wobei sowohl die Erdungs- als auch die Signalelektrode durch eine Vielzahl elektrisch leitender Fäden gebildet ist, die in das Kleidungsstück eingenäht und integriert sind, und wobei die zweiten Befestigungselmente (82) in direktem Kontakt mit der Erdungs- oder der Signalelektrode (31A, 32A) entsprechend verbunden sind.

15. Datenübertragungssystem nach Anspruch 14, wobei die Erdungs- und die Signalelektrode (3 1 A, 32A) jeweils in der Form von ringförmigen Bändern ausgebildet sind, die um einen Ärmel des Kleidungsstücks bezüglich der Länge des Ärmels mit Abstand voneinander angeordnet sind.

16. Datenübertragunssystem nach Anspruch 12, wobei das Gehäuse (11; 11A) für ein Versiegeln des elektrischen Schaltkreises wasserfest ausgebildet ist.

17. Datenübertragunssystem nach Anspruch 1, wobei das Gehäuse (11; 11A) in Form einer Platte zur Verfügung gestellt ist, die einen elektrischen Schaltkreis (28), der den ersten Modulator (15) realisiert, und die erste Signalübertragungsvorrichtung (16) und auch eine Batterie, die den Schaltkreis mit Leistung versorgt, verkapselt.

18. Datenübertragungssystem nach Anspruch 1, wobei
- die Empfangsvorrichtung (40) zusätzlich einen zweiten Modulator (47) zum Umwandeln zweiter Daten in ein zweites moduliertes Spannungssignal umfaßt, welches zwischen der Berührungselektrode (41) und der Schaltungserdung (49) angelegt wird;
- die Sendevorrichtung (10) ferner einen ersten Modulator (25) zum Umwandeln des zweiten modulierten Spannungssignals, welches über die Signalelektrode (32; 32A) detektiert wird, in die zweiten Daten umfaßt.

19. Datenübertragungssystem nach Anspruch 18, wobei die Sendevorrichtung (10) eine Steuerungsvorrichtung (14) zum Modifizieren der ersten Daten in Abhängigkeit von den zweiten Daten umfaßt.

20. Datenübertragungssystem nach Anspruch 1, wobei die ersten Daten einen Identifizierungscode (ID) eines einzelnen Benutzers umfaßt, der zu der Empfangsvorrichtung übertragen werden soll, und wobei die Empfangsvorrichtung einen ID-Speicher, der eine Vielzahl von Identifizierungscodes von Benutzern speichert, die einer entsprechenden Vielzahl von Benutzern zugewiesen sind, und eine Prüfvorrichtung umfaßt, die den ID der Benutzer im Vergleich mit der Vielzahl der den Benutzern zugewiesenen Identifizierungscodes prüft, so daß eine beabsichtigte Aktion erlaubt wird, die von dem Benutzer, der das Kleidungsstück trägt, angefragt wird.

21. Sendevorrichtung (10) für ein Datenübertragungssystem, das einen menschlichen Körper als Signalübertragungspfad nutzt, wobei die Sendevorrichtung (10) so ausgelegt ist, daß sie an einem menschlichen Körper getragen werden kann und folgendes umfaßt:
- Eine Erdungselektrode (31; 31A) und eine Signalelektrode (32; 32A), die in unmittelbarer Nähe des menschlichen Körpers angeordnet werden;
- einen Datenspeicher (13) zum Speichern erster zu übertragender Daten;
- eine erste Modulationsvorrichtung (15) zum Umwandeln der ersten Daten in ein erstes moduliertes Spannungssignal; und
- eine erste Signalsendevorrichtung (16), die das erste modulierte Spannungssignal über die Signalelektrode und die Erdungselektrode anlegt;
**dadurch gekennzeichnet, daß**
die Erdungs- und die Signalelektrode (31, 32; 31A, 32A) in einem Kleidungsstück (30) integriert sind, das so ausgelegt ist, daß es von einem Benutzer getragen werden kann, so daß ein elektrischer Pfad erzeugt wird, der sich durch einen Bereich des menschlichen Körpers für eine Signalübertragung von der Sendevorrichtung (10) zu einer Empfangsvorrichtung des Datenübertragunssystems erstreckt, wobei sowohl die Erdungselektrode (31; 31A) als auch die Signalelektrode (32; 32A) der Haut des Benutzers direkt gegenüberliegend und längs einer Oberfläche des Kleidungsstücks (30) mit Abstand voneinander angeordnet sind.

## Revendications

1. Système de transmission de données utilisant un corps humain comme ligne de transmission des signaux, ledit système comprenant un émetteur (10) conçu pour être porté sur un corps humain, et un récepteur (40) conçu pour être relié à un dispositif associé qui utilise les données émises depuis l'émetteur, ledit émetteur (10) comprenant :
- une électrode de terre (31 ; 31A) et une électrode de signal (32 ; 32A) qui sont placées à proximité immédiate du corps humain, et
- une mémoire de données (13) destinée à mémoriser des premières données à transmettre,
- un premier modulateur (15) destiné à convertir lesdites premières données en un premier signal de tension modulée, et
- un premier émetteur de signal (16) qui applique le premier signal de tension modulée aux bornes de ladite électrode de signal et de ladite électrode de terre,
ledit récepteur (40) comprenant :
- une masse de circuit (49) conçue pour être reliée à la terre,
- une électrode sensible au toucher (41) conçue pour un contact direct avec une partie du corps humain transportant ledit émetteur,
- un détecteur de signal (44) relié entre ladite électrode sensible au toucher et ladite masse de circuit afin de détecter ledit premier signal de tension modulée, et
- un démodulateur (45) destiné à convertir ledit premier signal de tension modulée en lesdites premières données,
**caractérisé en ce que**
ledit système comprend un vêtement (30) qui est conçu pour être porté par un utilisateur, et intègre lesdites électrodes de terre et de signal (31, 32 ; 31A, 32A) afin d'établir une ligne électrique passant par une partie du corps humain en vue d'une transmission de signal depuis ledit émetteur vers ledit récepteur, dans lequel à la fois ladite électrode de terre (31, 31A) et ladite électrode de signal (32 ; 32A) sont placées en relation de face à face rapprochée de la peau de l'utilisateur, et en relation espacée l'une par rapport à l'autre le long d'une surface dudit vêtement (30).

2. Système de transmission de données selon la revendication 1, dans lequel à la fois lesdites électrodes de terre et de signal (31, 32 ; 31A, 32A) sont portées sur le vêtement de façon à venir en relation face à face rapprochée de la peau de l'utilisateur.

3. Système de transmission de données selon la revendication 1, dans lequel chacune desdites électrodes de terre et de signal (31, 32 ; 31A, 32A) est formée d'une pluralité de fils électriquement conducteurs, et elle est cousue pour être intégrée au vêtement.

4. Transmission de données selon la revendication 3, dans laquelle chacune desdites électrodes de terre et de signal (31, 32 ; 31A, 32A) est prévue sous la forme d'un tissu réalisé avec les fils électriquement conducteurs, et est en doublure dudit vêtement.

5. Système de transmission de données selon la revendication 3, dans lequel chacune desdites électrodes de terre et de signal (31, 32 ; 31A, 32A) est intégrée audit vêtement afin de constituer une partie indispensable du vêtement.

6. Système de transmission de données selon la revendication 1, dans lequel chacune desdites électrodes de terre et de signal (31, 32 ; 31A, 32A) est formée d'un revêtement métallique déposé sur la surface du vêtement.

7. Système de transmission de données selon la revendication 1, dans lequel chacune desdites électrodes de terre et de signal (31, 32 ; 31A, 32A) est formée d'un revêtement métallique déposé sur un tissu qui est cousu sur le vêtement.

8. Système de transmission de données selon la revendication 2, dans lequel ladite électrode de terre (31 ; 31A) est située sur ledit vêtement plus près du pied de l'utilisateur que ladite électrode de signal (32 ; 32A) le long de la ligne électrique passant par la partie du corps humain.

9. Système de transmission de données selon la revendication 8, dans lequel ladite électrode de signal (32) est située sur une épaule du vêtement.

10. Système de transmission de données selon la revendication 3, dans lequel
les deux dites électrodes de terre et de signal (31, 32 ; 31A, 32A) sont portées sur le vêtement de façon à venir en relation face à face rapprochée de la peau de l'utilisateur,
ledit émetteur comprenant un boîtier (11) qui loge des circuits électriques (28) réalisant ledit premier modulateur (15), et ledit premier émetteur de signal (16), et qui est fabriqué séparément desdites électrodes de terre et de signal, ledit boîtier étant muni de bornes (71, 72) destinées à relier électriquement lesdits circuits électriques auxdites électrodes de terre et de signal,
ledit vêtement portant un fil de terre (33) et un fil de signal (34) qui s'étendent respectivement depuis lesdites électrodes de terre et de signal en vue d'une connexion avec lesdites bornes dudit boîtier, chacun desdits fils de terre et de signal étant formé d'un toron de fils électriquement conducteurs, et cousu sur ledit vêtement.

11. Système de transmission de données selon la revendication 10, dans lequel
ledit système comprend un moyen de couplage (70) par lequel ledit boîtier (11) est relié de façon amovible audit vêtement et en même temps lesdits circuits électriques sont reliés de façon amovible auxdites électrodes de terre et de signal (31, 32),
ledit moyen de couplage comprenant une pince à ressort (70) qui est supportée avec possibilité de rotation sur ledit boîtier pour être mobile entre une position de pincement et une position de libération, ladite pince étant munie desdites bornes (71, 72) qui sont isolées électriquement l'une par rapport à l'autre en vue respectivement d'une connexion avec lesdits fils de terre et de signal (33, 34) à ladite position de pincement.

12. Système de transmission de données selon la revendication 2, dans lequel
ledit émetteur (10) comprend un boîtier (10 ; 11A) qui loge des circuits électriques (28) réalisant ledit premier modulateur (15) et ledit premier émetteur de signal (16), et qui est fabriqué séparément desdites électrodes de terre et de signal, ledit boîtier étant muni de bornes (71, 72 ; 81) pour relier électriquement lesdits circuits électriques respectivement auxdites électrodes de terre et de signal,
ledit système comprenant un moyen de couplage (70 ; 81, 82) par lequel ledit boîtier est relié de façon amovible audit vêtement, et en même temps lesdits circuits électriques sont reliés auxdites électrodes de terre et de signal.

13. Système de transmission de données selon la revendication 12, dans lequel ledit moyen de couplage comprend une paire de premières attaches chacune étant composée de l'un d'une cavité (81) et d'un téton (82) formant un bouton pression en vue de monter ledit boîtier (11A) sur le vêtement (30), une paire de secondes attaches, chacune étant composée de l'autre de la cavité et du téton, lesdites premières attaches (81) étant fixées sur ledit boîtier et reliées aux bornes dudit premier émetteur de signal, et lesdites secondes attaches (82) étant fixées sur ledit vêtement et reliées respectivement de façon permanente auxdites électrodes de terre et de signal.

14. Système de transmission de données selon la revendication 13, dans lequel chacune desdites électrodes de terre et de signal est formée d'une pluralité de fils électriquement conducteurs cousus et intégrés au vêtement, et lesdites secondes attaches (82) sont maintenues respectivement en contact direct avec lesdites électrodes de terre et de signal (31A, 32A).

15. Système de transmission de données selon la revendication 14, dans lequel lesdites électrodes de terre et de signal (31A, 32A) sont respectivement en forme de bandes annulaires disposées autour d'une manche du vêtement, en relation espacée l'une par rapport à l'autre le long de la longueur de la manche.

16. Système de transmission de données selon la revendication 12, dans lequel ledit boîtier (11 ; 11A) est rendu étanche à l'eau pour rendre étanche lesdits circuits électriques.

17. Système de transmission de données selon la revendication 1, dans lequel ledit boîtier (11 ; 11A) est prévu sous la forme d'une plaque qui encapsule des circuits électriques (28) réalisant ledit premier modulateur (15) et ledit premier émetteur de signal (16), et également une pile (12) fournissant l'énergie aux circuits.

18. Système de transmission de données selon la revendication 1, dans lequel
ledit récepteur (40) comprend en outre un second modulateur (47) destiné à convertir des secondes données en un second signal de tension modulée qui est appliqué entre ladite électrode sensible au toucher (41) et ladite masse de circuit (49),
ledit émetteur (10) comprenant en outre un premier démodulateur (25) destiné à convertir ledit second signal de tension modulée, qui est détecté par l'intermédiaire de ladite électrode de signal (32 ; 32A), en lesdites secondes données.

19. Système de transmission de données selon la revendication 18, dans lequel
ledit émetteur (10) comprend un contrôleur (14) qui a pour fonction de modifier lesdites premières données conformément auxdites secondes données.

20. Système de transmission de données selon la revendication 1, dans lequel lesdites premières données comprennent un seul code d'identification d'utilisateur (ID) à transmettre audit récepteur, et ledit récepteur comprend une mémoire d'identificateurs ID mémorisant une pluralité de codes d'identification d'utilisateurs donnés respectivement à une pluralité d'utilisateurs, et un module de vérification qui vérifie les identificateurs ID des utilisateurs par comparaison avec la pluralité de codes d'identification d'utilisateurs affectés de façon à autoriser une action prévue requise par l'utilisateur portant ledit vêtement.

21. Emetteur (10) destiné à un système de transmission de données utilisant un corps humain comme ligne de transmission de signal, ledit émetteur (10) étant conçu pour être porté sur un corps humain et comprenant :
- une électrode de terre (31 ; 31A) et une électrode de signal (32 ; 32A) qui sont placées en proximité immédiate du corps humain,
- une mémoire de données (13) destinée à mémoriser des premières données à transmettre,
- un premier modulateur (15) destiné à convertir lesdites premières données en un premier signal de tension modulée, et
- un premier émetteur de signal (16) qui applique le premier signal de tension modulée aux bornes de ladite électrode de signal et de ladite électrode de terre,
**caractérisé en ce que** lesdites électrodes de terre et de signal (31, 32 ; 31A, 32A) sont intégrées à un vêtement (30) qui est conçu pour être porté par un utilisateur afin d'établir une ligne électrique passant par une partie d'un corps humain en vue d'une émission de signal depuis ledit émetteur (10) vers un récepteur dudit système de transmission de données, dans lequel à la fois ladite électrode de terre (31 ; 31A) et ladite électrode de signal (32, 32A) sont placées en relation face à face rapprochée de la peau de l'utilisateur, et en relation espacée l'une de l'autre le long d'une surface dudit vêtement (30).
